# EUROPEAN PATENT APPLICATION

(11) **EP 3 427 713 A1**
(43) Date of publication of application: **16.01.2019**
(21) Application number: 17762976.3
(22) Date of filing: 27.02.2017
(51) Int. Cl.: A61J 3/06, B41J 2/01

(54) **TABLET, TABLET PRINTING DEVICE, AND TABLET PRINTING METHOD**

(30) Priority: 07.03.2016 JP 2016043585
(71) Applicant: Freund Corporation, Tokyo 160-0023 (JP)
(72) Inventor: IMAI, Kiyoshi, Tokyo 160-0023 (JP); HACHIYA, Eiichi, Tokyo 160-0023 (JP); MOURI, Akira, Tokyo 160-0023 (JP)
(74) Representative: Hoffmann Eitle
(86) International application number: PCT/JP2017/007470
(87) International publication number: WO 2017/154636

(57) **Abstract**

A tablet 3 has a first layer 51 printed using visible ink and a second layer 52 printed using invisible ink. The first layer 51 includes characters, a trademark, or the like printed using the visible ink, which can be visually confirmed by an administrator in charge or a tablet user. The second layer 52 includes a barcode or the like printed using the invisible ink, which cannot be visually confirmed by the administrator or the tablet user. The second layer 52 is visualized by black light or the like so as to be made readable. The tablet 3 is subjected to print processing by a tablet printing device 1 provided with conveying disks A to C conveying the tablet 3 while sucking and supporting a side surface 7 of the tablet 3. The first layer 51 and the second layer 52 are printed by inkjet heads 4 and 5, respectively.

## Description

### Technical Field

The present invention relates to a tablet on the surface of which a product number, a product name, a trademark, a barcode, or the like is printed and, more particularly, to a tablet on the surface of which inkjet print processing is applied using visible ink and invisible ink, and a printing device and method for the tablet.

### Background Art

In recent years, in hospitals, pharmacies, and the like, various drugs are subject to one-dose packaging for individual patients. In this case, in pharmacies and nursing/caring fields, etc., (hereinafter, abbreviated as "pharmacy or the like"), it is necessary to check whether a drug package contains a predetermined number of prescribed drugs without mistake, whether the package is given to a right recipient, and the like. Further, in many cases, generic drugs using off-patent ingredients that contain the same active ingredients differ in tablet shape. Thus, there are cases where tablets differ in shape are given to different recipients, although the recipients are prescribed the same medicine. Therefore, in pharmacies or the like, drugs are managed while confirming displays on tablets to prevent erroneous dispensing for the recipient of the drug.

On the other hand, a product number, a product name, a trademark or the like is written on the surface of a tablet or a capsule for identification of a product and prevention of accidental ingestion. Such a display on the tablet is made by engraving at tabletting or print processing such as transfer printing or inkjet printing. In these methods, since the print processing by the inject printing is capable of applying printing to the tablet surface in a non-contact manner, it is thus less susceptible to powder adhering to irregularity of the tablet surface and excellent in sanitation. Thus, in recent years, various devices have been proposed as an inkjet type tablet printing device (for example, in Patent Document 1).

### Citation List

### Patent Document

Patent Document 1: Japanese Patent No. 5,281,009
Patent Document 2: JP 2006-89741 A

### Disclosure of the Invention

### Problems to be Solved by the Invention

However, when a display item such as drug name or volume is small, it inevitably becomes hard to read even if it can be printed clearly on the tablet surface. In particular, when a large number of tablets with a small display item are included in one dose package, it is difficult to definitely confirm them without error. Thus, a task burden on an administrator in charge in a pharmacy or the like is increased due to one-dose packaging. In this case, when the size of a tablet is increased, the printing surface is correspondingly increased, with the result that printing becomes easy to read. However, since an excessively large tablet is difficult to swallow, the size of the tablet is unavoidably subject to limitations. That is, the size (printable area) of the tablet having an easily swallowable form is restricted, thus making it difficult to write many information items (characters or marks) on the tablet surface in an easy-to-read manner.

For a tablet, the quality of visibility may lead to erroneous dispensing by pharmacists or accidental ingestion of drug users such as patients and is thus a major problem for both parties and, thus, in the modern age in which elderly persons are increasing, improvement of the visibility is an urgent task. Under the circumstances where various prescriptions exist, manual inspection by the human eye (visual inspection) is not sufficient as a countermeasure against the errors. Further, in the visual inspection, it is difficult to keep confirmation records, so that traceability is not ensured. Further, recently, the shape of the drug has been diversified like generic drugs having a variety of shapes or sizes but same in ingredient, making it difficult to improve inspection of drugs without involving an increase in task burden on the administrators.

### Means for Solving the Problems

A tablet according to the present invention has a first layer printed using visible ink and a second layer printed using invisible ink. In the present invention, the first layer includes characters, a trademark, or the like printed using the visible ink, which can be visually confirmed by the administrator or a tablet user. The second layer includes a barcode or the like printed using the invisible ink, which cannot be visually confirmed by them. The second layer is visualized by black light or the like so as to be made readable.

In the tablet, the first and second layers overlapping each other may be disposed on the tablet. Further, the first and second layers may be formed at least one of the front and back surfaces of the tablet. The first and second layers may each be formed on the side surface of the tablet.

A tablet printing device according to the present invention includes a conveying unit conveying a tablet while sucking and supporting the side surface of the tablet and a printing unit disposed in proximity to the conveying unit and capable of applying print processing at least to one of the front and back surfaces of the tablet. The printing unit forms a first layer printed using visible ink and a second layer printed using invisible ink on the tablet.

In the present invention, the first and second layers are printed with the side surface of the tablet sucked and supported, so that inspection or print processing of each layer can be performed with the front and back surfaces of the tablet exposed. Further, on the conveying unit, the first and second layers can be printed on both the front and back surfaces of the tablet, so that the layers can be printed on both the front and back surfaces without reversing the tablet.

In the tablet printing device, a conveying disk formed into a disk shape may be used as the conveying unit, and the conveying disk may have suction parts formed in the end surface thereof in the peripheral direction and each sucking the side surface of the tablet.

On the other hand, a tablet printing method according to the present invention includes conveying a tablet while sucking and supporting the side surface of the tablet, and forming a first layer printed using visible ink and a second layer printed using invisible ink at least on one of the front and back surfaces of the conveyed tablet.

In the present invention, the first and second layers are printed with the side surface of the tablet sucked and supported, so that inspection or print processing of each layer can be performed with the front and back surfaces of the tablet exposed. Further, the first and second layers can be printed on both the front and back surfaces of the tablet, so that the layers can be printed on both the front and back surfaces without reversing the tablet.

### Advantages of the Invention

According to the present invention, the first layer can be visually confirmed, and the second layer which cannot be seen by the human eye can be mechanically confirmed by an inspection device or the like, so that consistency between actual drugs and prescription can be double-checked by the administrator and a machine. This can improve accuracy of dispensing inspection to thereby reduce dispensing error and can also reduce a task burden on the administrators in pharmacies or the like.

According to the tablet printing device and method of the present invention, the first and second layers are printed with the side surface of the tablet sucked and supported, so that inspection or print processing of each layer can be performed with the front and back surfaces of the tablet exposed. Further, the first and second layers can be printed on both the front and back surfaces of the tablet, so that the layers can be printed on both the front and back surfaces without reversing the tablet.

### Brief Description of the Drawings

[FIG. 1] An explanatory view illustrating the entire configuration of a tablet printing device according to an embodiment of the present invention.
[FIG. 2] An explanatory view illustrating an example of a tablet according to the present invention.
[FIG. 3] Explanatory views each illustrating an example of a dispensing form.
[FIG. 4] A block diagram illustrating the configuration of a tablet inspection system.

### Best Mode for Carrying Out the Invention

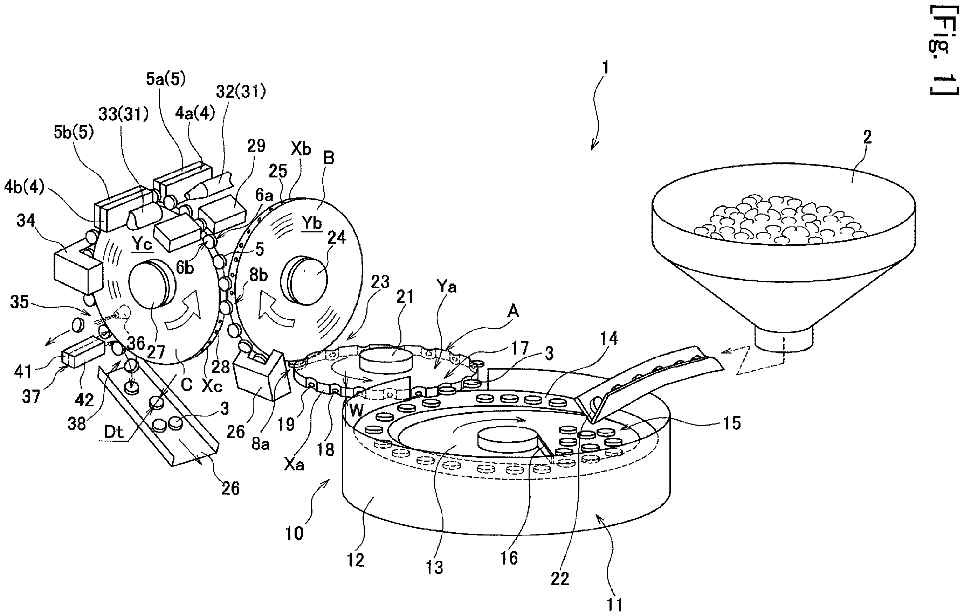

The object of the following embodiment is to improve inspection accuracy for prescription drugs to reduce erroneous dispensing and to reduce a task burden on an administrator in charge in pharmacies or the like. FIG. 1 is an explanatory view illustrating the entire configuration of a tablet printing device 1 according to an embodiment of the present invention. The tablet printing device 1 of FIG. 1 sucks and conveys tablets 3 fed from a hopper 2 one by one with three conveying disks A, B, and C formed into a disk shape. Then, inkjet heads 4 and 5 are used to perform two-layer print processing for each of front and back surfaces 6a and 6b of the tablet 3.

In the tablet 3 according to the present invention, a first layer 51 using visible ink and a second layer 52 using invisible ink 52 are printed on each of the front and back surfaces 6a and 6b. The first layer 51 is printed by the inkjet head 4, and the second layer 52 is printed by the inkjet head 5. In this case, characters representing a product name or the like is printed on the first layer 51, and a barcode (one-dimensional barcode, two-dimensional barcode) for management (manufacturing management, product management, medication management, etc.) or the like is printed on the second layer 52. As required, it is possible to omit print processing on one of the front and back surfaces 6a and 6b, to add another inkjet head for applying print processing to a side surface 7 of the tablet 3, or to apply multilayer print processing of three or more layers. As a matter of course, the visible or invisible ink to be used in print processing is edible.

In the tablet printing device 1, the tablets 3 are transferred from the conveying disk A (tablet supply disk) to the conveying disk B (first conveying disk) at a tablet delivery part 8a. Further, at a tablet delivery part 8b, the tablets 3 are transferred from the conveying disk B to the conveying disk C (second conveying disk) . In the conveying disks A and C, the side surface 7 of each tablet 3 is sucked to and supported on end surfaces (tablet holding parts) Xa and Xc of the respective disks. In the conveying disk B, the front surface 6a or back surface 6b of each tablet 3 is sucked to and supported on an end surface (tablet holding part) Xb of the disk. The tablet 3 is conveyed to the inkjet heads 4 and 5 in a standing state (standing posture) where the side surface 7 is directed in the vertical direction. In the tablet printing device 1, the front and back surfaces 6a and 6b of the tablet 3 are subjected to printing at the same time by the inkjet heads 4 and 5. Then, only good products are discharged outside the device through a good product discharge part 38.

As illustrated in FIG. 1, the tablet printing device 1 is provided with the hopper 2, a rotary feeder (tablet feeder) 11, and the conveying disk A as a tablet supply unit 10. The hopper 2 stores the tablets 3 and feeds them to the rotary feeder 11. The rotary feeder 11 feeds the tablets 3 received from the hopper 2 to the conveying disk A. The conveying disk A sucks and conveys the tablets 3 from the rotary feeder 11 to the tablet delivery part 8a. The rotary feeder 11 is a so-called vibrationless rotary parts feeder and has a configuration in which a rotary disk 13 and an annular rotary plate 14 are coaxially provided in a cylindrical casing 12. The annular rotary plate 14 is disposed immediately inside the casing 12. The rotary disk 13 is disposed inside the annular rotary plate 14 in an inclined state. A part of the outer periphery of the rotary disk 13 has the same height as the annular rotary plate 14 and the part serves as a communication part 15 between the rotary disk 13 and the annular rotary plate 14. The communication part 15 is provided with a guide plate 16 for guiding the tablets on the rotary disk 13 toward the annular rotary plate 14 side.

The casing 12 is partially cut out. The conveying disk A is disposed in such a manner that the outer peripheral portion thereof faces the cut out portion of the casing 12 to form a tablet acquisition part 17. The end surface Xa of the conveying disk A has a width W smaller than a maximum diameter Dt of the tablet 3. The end surface Xa has a plurality of circular suction holes (suction parts) 18 which are arranged at equal intervals in the peripheral direction. The suction holes 18 are connected to a suction device (not illustrated) such as a vacuum pump. A recessed part 19 in which the tablet 3 is introduced is formed around each of the suction holes 18 formed in the end surface Xa. The conveying disk A is disposed with a disk surface Ya thereof being substantially horizontal and is rotated about a vertically extending rotary shaft 21 in a direction denoted by the arrow by a drive source (not illustrated) . As illustrated in FIG. 1, the conveying disks B and C provided at stages subsequent to the conveying disk A are disposed with disk surfaces Yb and Yc thereof being upright. On the other hand, the conveying disk A is disposed with the disk surface Ya being horizontal like a turn table. At the tablet acquisition part 17, the tablet 3 is sucked at its side surface 7 to the suction hole 18 of the conveying disk A and then conveyed to the conveying disk B in a horizontal posture (in a state where the front and back surfaces 6a and 6b face upward and downward in the vertical direction).

In the rotary feeder 11, the tablet 3 supplied from the hopper 2 is fed from a tablet supplying part 22 onto the rotating rotary disk 13. The tablet 3 on the rotary disk 13 moves in the peripheral direction with rotation of the rotary disk 13 and is then guided by the guide plate 16 to the annular rotary plate 14 being rotated at the same speed as the rotary disk 13. The tablet 3 on the annular rotary plate 14 moves in the peripheral direction with rotation of the annular rotary plate 14 to be fed to the tablet acquisition part 17. The tablet 3 fed to the tablet acquisition part 17 faces the end surface Xa of the rotating conveying disk A. At this time, if the tablet 3 coincides in posture and timing with the suction hole 18, it is sucked to the suction hole 18 while being fitted in the recessed part 19. That is, the tablet 3 sucked in a proper posture is conveyed by the conveying disk A to a tablet feeding part 23 which is a contact point with the conveying disk B. On the other hand, if the tablet 3 does not coincide in posture and timing with the suction hole 18 and thus is not sucked to the suction hole 18, it stays on the rotating rotary disk 13. That is, any tablet that does not assume a suction posture returns to the rotary feeder 11 and is then automatically conveyed toward the tablet acquisition part 17 once again (automatic return/automatic retry).

In the tablet printing device 1, the tablet 3 is sucked and conveyed from the rotary feeder 11 to the tablet feeding part 23 using the conveying disk A. By this suction conveyance, the tablet 3 can be picked up and fed to the conveying disk B in a guideless configuration where a guide member matching a tablet size is not used. In recent years, drugs, such as generic drugs, having different size or shape while maintaining the same ingredient are widely accepted in the market. Further, there are many cases that tablets of various sizes having different prescriptions exist although they are the same drugs. However, in a configuration where the guide member matching the tablet size provided between the tablet feeder and the tablet feeding part 23 is used to align the tablets 3 and guide them to the tablet feeding part 23, the guide member needs to be replaced by a proper one every time the size of the tablet is changed. Thus, even in the case of tablets of the same ingredient like the generic drug, when the sizes thereof are different, component replacement is required. Further, every time the component is replaced, the operation of the device must be interrupted to perform replacement/cleaning work, taking much labor and increasing processing time.

In contrast to this, in the tablet printing device 1, the tablet 3 is fed to the tablet feeding part 23 not using the guide member, but by the conveying disk A. As a result, the tablet can be fed to the subsequent stage in a proper posture irrespective of the tablet size as long as the tablet can be sucked to the disk, thereby flexibly dealing with tablets of various sizes. Thus, tablets of the same ingredients having various sizes can be conveyed in a mixed state, thereby significantly improving the processing efficiency. Further, the tablet printing device 1 performs print processing while the conveying disks B and C arranged at subsequent stages perform suction conveyance, so that it is also possible to achieve desired printing irrespective of the tablet size. That is, using the conveying disk A allows achievement of effective print processing exerting the features of the device at the maximum. Further, the tablet printing device 1 is not provided with a conveying guide, so that exchange or cleaning thereof need not be performed, thereby reducing man-hours for device maintenance. Additionally, the arrangement pitch of the suction holes 18 is previously determined, so that it is possible to prevent a large number of tablets exceeding the processing capacity from being fed to the tablet feeding part 23, which in turn prevents problems of jamming of tablets.

The tablet 3 is conveyed to the tablet feeding part 23 with rotation of the conveying disk A while maintaining the horizontal posture. Then, at the tablet delivery part 8a, the tablet 3 is handed over from the conveying disk A to the conveying disk B. At the tablet feeding part 23, the end surface Xa of the conveying disk A is disposed so as to adjacently face the end surface Xb of the conveying disk B in an orthogonal state, and the tablet delivery part 8a is formed between the conveying disks A and B. The conveying disks A, B, and C are synchronously driven so that the conveying speed of the tablet 3 is made constant considering transfer of the tablet therebetween. The conveying disk B is rotated about a rotary shaft 24 in a direction denoted by the arrow by a drive source (e.g., an electric motor; not illustrated). The end surface Xb of the conveying disk B has a flat surface with no projection. The end surface Xb has a plurality of circular suction holes (suction parts) 25 which are arranged at equal intervals in the peripheral direction. Like the suction holes 18, the suction holes 25 are connected to a suction device (not illustrated) such as a vacuum pump. The tablet 3 fed to the tablet feeding part 23 is sucked to the end surface Xb of the conveying disk B by the suction hole 25. At this time, the tablet 3 is held to the end surface Xb with one of the front and back surfaces 6a and 6b sucked thereto.

A side surface inspection device 26 is disposed near the conveying disk B. The side surface inspection device 26 is used for inspecting a state (presence/absence of cracking and chipping) of the side surface 7 of the tablet 3 sucked to and supported by the conveying disk B (side surface inspection). In the tablet printing device 1, a camera is used as the inspection device for inspecting the outer appearance or a printed state of the tablet. An image photographed by the inspection device is sent to a controller (not illustrated), where determination of nondefective/defective is made. A light and a pair of prisms are provided in the photographing range of the camera used for the side surface inspection device 26. The pair of prisms are disposed so as to face the tablet side surface. The camera photographs the state of the tablet side surface illuminated by the light 180 degrees at a time by two prisms. The side surface inspection device 26 can not only inspect the outer appearance of the tablet 3, but also measure the thickness thereof, so it can also determine a dimensional error. A tablet 3 in which any abnormality is detected is recognized as a defective and discharged through a defective product discharge part 35 provided at a stage subsequent to the conveying disk C without being subjected to print processing.

The tablet 3 sucked to the end surface Xb of the conveying disk B is put into an upright posture (a state where the side surface 7 is directed in the vertical direction) from the horizontal posture with rotation of the conveying disk B, subjected to the inspection by the side surface inspection device 26, and fed to the tablet delivery part 8b. The conveying disk C is disposed at the tablet delivery part 8b. The conveying disk C is rotated about a rotary shaft 27 in a direction denoted by the arrow by a drive source (not illustrated) . The conveying disks B and C are disposed such that the rotary shafts 24 and 27 thereof are orthogonal to each other and are synchronously driven so as to convey the tablets at the same speed. As in the conveying disk B, an end surface Xc of the conveying disk C also has a flat surface with no projection. A width W of the end surface Xc is also smaller than the maximum diameter Dt of the tablet 3. The end surface Xc of the conveying disk C also has circular suction holes (suction parts) 28 which are arranged at equal intervals in the peripheral direction. The end surfaces Xb and Xc of the respective conveying disks B and C are disposed so as to adjacently face each other at the tablet delivery part 8b in an orthogonal state.

The tablet 3 is sucked to the end surface Xb of the conveying disk B at the tablet delivery part 8a and conveyed to the tablet delivery part 8b with rotation of the conveying disk B. The tablet 3 conveyed to the tablet delivery part 8b is sucked to the end surface Xc of the conveying disk C there and is then handed over to the conveying disk C side. In this case, at the conveying disk B side, suction force is imparted to the suction holes 25 until they reach a position at which both the disks B and C are closest to each other at the tablet delivery part 8b. The tablet 3 is sucked to the suction hole 28 of the opposing conveying disk C from the suction hole 25 losing the suction force at the disk closest approach position, and the tablet 3 is transferred to the conveying disk C.

The tablet 3 is sucked to the conveying disk B at one of its front and back surfaces 6a and 6b, so that the side surface 7 of the tablet 3 faces the end surface Xc of the conveying disk C at the tablet delivery part 8b. Thus, at the conveying disk C side, the side surface 7 of the tablet 3 is sucked and, accordingly, the tablet 3 is held to the end surface Xc in a standing state. Clearances between the conveying disks A, B, and C at the tablet delivery parts 8a and 8b can be changed in dimension according to the size of the tablet 3 and is automatically adjusted by inputting the tablet size through a control panel.

A print surface inspection device 29 is disposed at a stage subsequent to the tablet delivery part 8b so as to be in proximity to the conveying disk C. The print surface inspection device 29 inspects states of the front and back surfaces 6a and 6b of the tablet 3 sucked to and supported by the conveying disk C (print surface inspection) . In the case of a scored tablet 3, the position of the scoring line is also detected by the print surface inspection device 29. As in the case of the side surface inspection device 26, a tablet 3 for which defective outer appearance is detected is recognized as a defective and discharged through the defective product discharge part 35 without being subjected to print processing.

The inkjet heads 4 and 5 are provided at a stage subsequent to the print surface inspection device 29. In the tablet printing device 1, a powder removing device 31 is provided at a former stage of the inkjet heads 4 and 5. The powder removing device 31 blows compressed air to the tablet 3 from a nozzle 32 to remove powder adhering to the tablet surface immediately before printing. The powder blown off from the tablet surface is collected by a suction tube 33. Powder such as drug adheres to the surface of a tablet formed by a tabletting machine. If print processing is applied to the tablet surface without removing the powder, the print may be erased from the tablet surface together with the powder, or print blurring may occur due to bleeding. To cope with this problem, in the tablet printing device 1, the powder removing device 31 is disposed immediately before the inkjet heads 4 and 5. As a result, powder adhering to the surface of the tablet 3 is removed. In addition, print processing can be performed before generation of new powder. Thus, it is possible to prevent erasing or bleeding of the print due to the powder adhering to the tablet surface, allowing clear and high-quality print processing to be obtained.

After clearance of the tablet surface by the powder removing device 31, the front-stage inkjet head 4 is used to apply first layer print processing to the front and back surfaces 6a and 6b of the tablet 3 with the visible ink, and the rear-stage inkjet head 5 is used to apply second layer print processing to the front and back surfaces 6a and 6b of the tablet 3 with the invisible ink. During the print processing, the tablet 3 is supported with the side surface 7 sucked, and thus the entire front and back surfaces 6a and 6b are exposed. Thus, in the tablet printing device 1, print processing can be applied to the entire front and back surfaces 6a and 6b of the tablet 3. Particularly, in applying print processing to the front and back surfaces 6a and 6b, an area where printing cannot be applied is not generated in the peripheral edge of the tablet.

In the inkjet heads 4 and 5, inkjet heads 4a and 5a for front surface and inkjet heads 4b and 5b for back surface are disposed so as to face each other at the same position, so that print processing can be applied to the front and back surfaces 6a and 6b of the tablet 3 at the same time by using the inkjet heads 4 and 5. Further, the direction of characters to be printed on the front surface 6a of the tablet 3 and the direction of characters to be printed on the back surface 6b thereof can be made to coincide with each other according to the direction of the scoring line detected by the print surface inspection device 29. Although the inkjet heads 4a and 5a for front surface and the inkjet heads 4b and 5b for back surface are disposed so as to face each other at the same position, they may be displaced from each other. Further, another inkjet head may be provided for multicolor or multilayer printing.

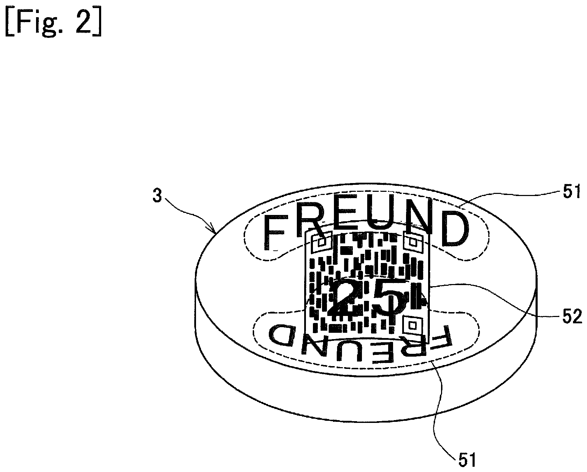

FIG. 2 is an explanatory view illustrating an example of the tablet 3 that has been subjected to print processing. Although only the front surface 6a is illustrated in FIG. 2, the same print processing is applied to the back surface 6b. Two layers (first layer 51 and second layer 52) are superimposed on the front and back surfaces 6a and 6b of the tablet 3, whereby, print information items are layered. As illustrated in FIG. 2, the first layer 51 includes characters representing a product name, an active ingredient, a tablet provider, a volume, or the like. For example, in FIG. 2, a volume ("25" = 25 mg) and a provider name ("FREUND") are printed as the first layer 51. The characters of the first layer 51 are printed using the visible ink, thus allowing the administrator or a tablet user to visually confirm them.

On the other hand, the second layer 52 includes a barcode or the like. For example, in FIG. 2, a two-dimensional barcode is printed. The barcode of the second layer 52 is printed using the invisible ink, so that the administrator or the tablet user cannot visually confirm it. Thus, a complicated printing like the barcode is not usually visible on the tablet surface, so that tablet design is not impaired by the second layer 52. In this case, the second layer 52 is visualized by a black light (long-wave ultraviolet ray in the visible region), an ultraviolet ray in the invisible region, a laser light, or the like so as to be made readable. That is, the first layer 51 is visible by the human eye, while the second layer 52 cannot be seen by the human eye without using a machine such as an inspection device. The same content (active ingredient name, volume, or the like) as the first layer 51 is printed on the second layer 52 together with data associated with print processing (e.g., serial number added to each tablet).

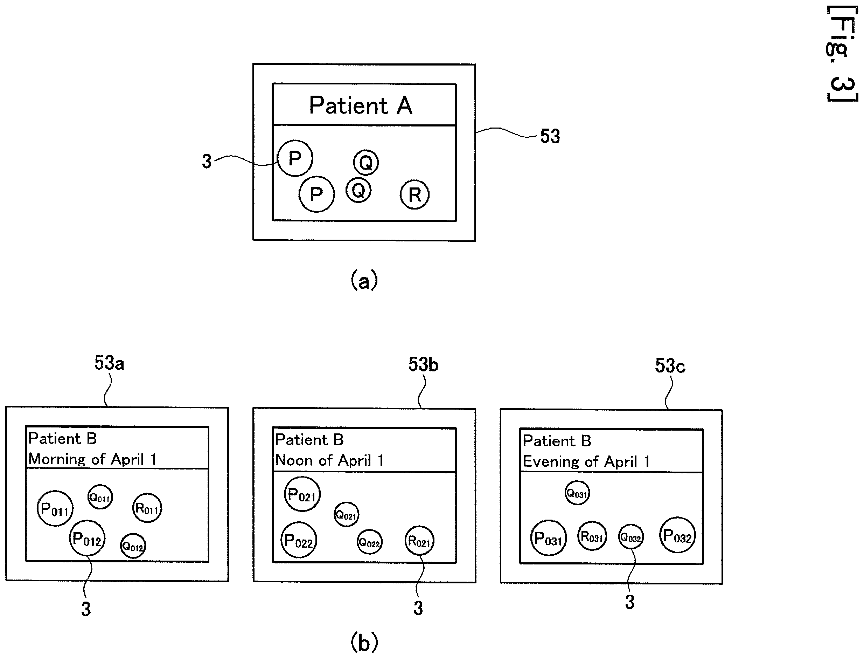

When preparing such a tablet 3, inspection of the first layer 51 is performed by visual observation, and at the same time, inspection of the second layer is also performed using a machine. That is, consistency between actual drugs and prescription is confirmed for each tablet by using both the character recognition and the barcode. FIGS. 3A and 3B are explanatory views each illustrating an example of a dispensing form. For example, assume that drugs prescribed for a patient A include "tablet P × 2", "tablet Q × 2", and "R", and that they are subject to one-dose packaging as illustrated in FIG. 3A (package 53). In this case, the dispenser has to carry out a visual dispensing work for the package 53 containing five tablets and which is 14 days' worth of medication (three packages per day), namely 43 packages in total, which is very troublesome work, and a check error is apt to occur.

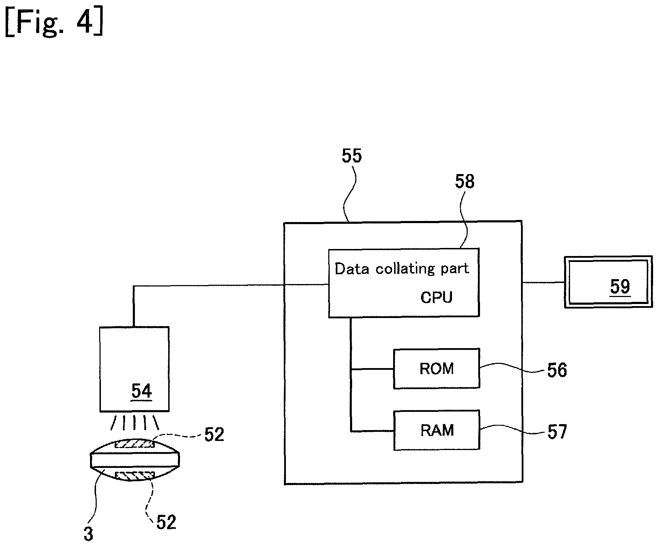

In contrast to this, in the case of the package 53 using the tablets 3 according to the present invention, inspection can be performed in a short time only by using an inspection device 54. FIG. 4 is a block diagram illustrating the configuration of an inspection system for the tablet 3. In the inspection system, first, an inspection device 54 is used to irradiate the tablet 3 with an identifying light such as a black light to visualize the second layer 52 and to read the barcode printed on the tablet 3. Then, information (active ingredient name, volume, serial number, or the like) represented by the barcode is transmitted to a computer-based data collating device 55.

The data collating device 55 is provided with a ROM 56 and a RAM 57 storing dispensing data and a data collating part 58. The data collating part 58 collates inspection data received from the inspection device 54 with the dispensing data stored in the ROM 56 or the like. In this example, prescription for the patient A is stored as the dispensing data. The data collating part 58 compares the dispensing data with actual inspection data and determines "correct (OK)" when they match each other. On the other hand, when the dispensing data and actual inspection data do not match each other, the data collating part 58 determines "not correct (NG) ". The determination result is notified to the administrator or the like by a display device 59 with an image or voice.

As described above, by using the tablet 3 according to the present invention, consistency between prescription and actual drugs is double-checked by both the eyes of the administrator and the inspection device 54. In this case, the inspection device 54 determines whether or not the dispensing is correct by the machine, so that the determination accuracy thereof is higher than visual observation. Thus, inspection accuracy is significantly improved as compared with a case where dispensing is managed only by visual observation, allowing determination accuracy on whether or not the dispensing is correct to be dramatically improved. This can significantly reduce dispensing error and can also reduce a task burden on the administrator. In particular, when a large number of tablets are subject to one-dose packaging, both the inspection accuracy and task burden are significantly improved.

Further, the inspection data can be stored reliably, so that traceability can be ensured. Furthermore, medication data can be managed in the form of digital data, so that E-medicine notebook function can be added. In addition, tablet data can be printed in a non-visible manner, so that it is possible, by using the machine, to discriminate between an active drug and its counterpart placebo which looks almost like the real counterpart, thereby allowing the technique according to the present invention to be effectively used also for determination of the placebo.

On the other hand, as described above, a serial number can be added to each tablet, so that it is possible to make distinction between individual tablets or individual packages. For example, as illustrated in FIG. 3B, when drugs P (× 2), Q (× 2), and R are prescribed for a patient B, unique numbers are added to the tablets 3 in each package 53 as follows: P₀₁₁, P₀₁₂, Q₀₁₁, Q₀₁₂, R₀₁₁ (package 53a: for morning of April 1) ; P₀₂₁, P₀₂₂, Q₀₂₁, Q₀₂₂, R₀₂₁ (package 53b: for noon of April 1), and P₀₃₁, P₀₃₁, Q₀₃₁, Q₀₃₂, R₀₃₁ (package 53c: for evening of April 1). The patient B is required to photograph the package 53 with a smartphone or the like every time when taking drugs and to present or transmit the photograph periodically. This allows the medication side or nursing/caring side to check whether the patient takes predetermined drugs at predetermined timings. At this time, when only an image of the drugs of the same number is transmitted, there is a possibility that the patient B does not take the drugs correctly. In this case, immediate measures can be taken. When an image is not transmitted, measures including making contact with the patient can be taken.

Further, for example, dosing data like "patient B: morning of April 1" can be printed on the second layer 52 of the tablet 3. In this case, when a nurse or a caregiver collates and confirms the dosing data at medication using a mobile terminal having a configuration obtained by making compact the system of FIG. 4, it is possible to prevent medication error (e.g., drugs are mistakenly given to a wrong patient, or a patient is given wrong medication) . This reduces a burden not only on the administrators or tablet users, but also on the on-site nursing/caring staff.

A print inspection device 34 is provided at a stage subsequent to the inkjet heads 4 and 5. The print inspection device 34 is provided for checking a result of printing performed by the inkjet heads 4 and 5. When a printing defect is detected by the print inspection device 34, the corresponding tablet is discharged through the defective product discharge part 35 provided at a stage subsequent to the print inspection device 34. The defective product discharge part 35 is provided with a jet nozzle 36 that blows compressed air. A tablet 3 determined to have a defect in outer appearance or printing is blown off from the conveying disk C by the air blown from the jet nozzle 36 to be removed.

A drying/cooling device 37 is provided at a stage subsequent to the defective product discharge part 35. When the tablet 3 is conveyed to the good product discharge part 38 while the print surface thereof is yet to be dried, the ink may adhere to a carrying-out path 39 or print blurring may occur. In order to cope with this problem, in the tablet printing device 1, the drying/cooling device 37 is provided at a stage preceding the good product discharge part 38 so as to dry the print surface and solidify the ink. The drying/cooling device 37 is provided with a heating nozzle 41 and a cooling nozzle 42. In the drying/cooling device 37, the solvent of the ink is vaporized by hot air from the heating nozzle 41, and then the ink is cooled by cooling air from the cooling nozzle 42 to the melting point or lower thereof for solidification. Then, the print is fixed to the tablet surface, and only tablets 3 determined to be non-defective are discharged through the good product discharge part 38. As a result, it is possible to prevent the tablets determined to be non-defective from being stained by ink adhering to the carrying-out path 39 and to prevent print blurring due to rubbing of the print surface when the tablets 3 is rolling down the carrying-out path 39, thereby improving product quality and yield.

As described above, in the tablet printing device 1 according to the present invention, print processing is performed while sucking and supporting the side surface 7 of the tablet 3, so that the entire front and back surfaces 6a and 6b of the tablet 3 are not covered with the guide or the like for supporting the tablet. This allows inspection or print processing to be performed in a state where the surface to be printed is exposed, and prevents an area where inspection or print processing cannot be applied from being generated at the peripheral portion on the front and back surfaces of the tablet. Further, both the front and back surfaces can be subjected to printing without involving reversal of the tablet 3, thereby eliminating the risk of ink transfer or print blurring associated with the reversal of the tablet. In addition, the both surfaces of the tablet 3 can be subjected to printing at the same time, so that the directions of characters printed on the front and back surfaces 6a and 6b can be made to coincide with each other.

Thus, according to the tablet printing device 1, desired print can be efficiently applied to the entire front and back surfaces, thereby achieving improvement in the degree of freedom of print design, and reduction in printing time and device size. Further, in the tablet printing device 1, the tablet 3 is conveyed to the position of the inkjet heads 4 and 5, not using a conveying tool such as a magazine, but in a state of being sucked to and supported by the conveying disk C. Thus, in the inkjet heads 4 and 5, the tablet 3 can pass just near the head nozzle, allowing the distance between the head and the tablet to be reduced as much as possible. Thus, printing accuracy with respect to the front and back surfaces can be enhanced to thereby realize the tablet printing enhanced in quality.

Furthermore, in the inkjet heads 4 and 5 of the tablet printing device 1, an ink ejection amount is adjusted according to the shape of the front and back surfaces 6a and 6b, so that even when the print surface is a curved surface, printing can be performed without distortion. For example, in the case of a tablet whose front and back surfaces are spherical, there occurs a difference between the distance between the center portion of the tablet and the head nozzle and the distance between the peripheral portion thereof and the head nozzle. Therefore, if the tablet is printed in the same way at the center and peripheral portions, distortion may occur at the peripheral portion. Thus, in the tablet printing device 1, the ejection amount of the head nozzle is adjusted according to shape information of the tablet so as to achieve easy-to-see printing free from distortion. While the shape information of the tablet can be input from a control panel of the device, thickness or outer diameter data of the tablet 3 measured by the side surface inspection device 26 can also be exploited.

The present invention is not limited to the above-described embodiment and may be variously changed within the scope of the invention.

For example, although circular tablets are subjected to print processing in the above embodiment, the tablet printing device according to the present invention may be applied not only to the circular tablets, but also to various types of tablets such as oblong tablets, caplets, and polygon-shaped tablets. Further, print processing can be applied not only to the tablets, but also to capsules (hard, soft) . Thus, the "tablet" in the present invention is a concept that includes not only so-called circular tablets, but also tablets or capsules of various shapes.

Further, the shape of the suction hole formed in the conveying disk of the above embodiment is not limited to a circular shape, but may be an elliptical or polygonal shape. Further, a curved suction groove having an inner peripheral surface matching the tablet outer shape may be formed together with the suction hole. In this case, as the shape of the suction groove, various shapes such as a V- or U-like shape or a quadrangular shape may be adopted. For example, a substantially V-shaped groove may be formed in the tablet feeding disk so as to correspond to odd-shaped tablets such as triangular tablets. The most common shape of the tablet is a disk shape, and thus the groove is desirably formed into a curved shape to which the tablets slightly different in size can be fitted. A recessed part similar to that of the conveying disk A may be formed in the end surfaces Xb and Xc of the conveying disks B and C, and conversely, the end surface Xa of the conveying disk A may be formed into a flat surface like the conveying disks B and C.

Furthermore, when another inkjet head is added to apply print processing to the side surface 7, a tablet provider name can be printed on the side surface 7, so that the active ingredient name or volume on the front and back surfaces 6a and 6b can be made large. That is, a printable range is expanded not only to the front and back surfaces as in the conventional case, but also to the side surface, so that printable area is increased, and correspondingly, the active ingredient name or the like can be made larger, or the number of characters to be printed can be increased, whereby visibility is enhanced. Further, a barcode can be printed on the side surface 7 and, in this case, printing on the side surface 7 can be performed using visible or invisible ink. That is, the second layer 52 can be formed alone on the side surface 7. The first and second layers 51 and 52 need not overlap each other, but may be formed independently from each other on the front surface 6a, back surface 6b, or side surface 7.

### Industrial Applicability

The present invention may be applied to printing for medical tablets, but also for food such as confectionery having a tablet shape.

### Reference Signs List

- 1:: Wiper system
- 2:: Brushless motor
- 1:: Tablet printing device
- 2:: Hopper
- 3:: Tablet
- 4:: Inkjet head (for first layer: printing unit)
- 4a:: Inkjet head for front surface (printing unit)
- 4b:: Inkjet head for back surface (printing unit)
- 5:: Inkjet head (for second layer: printing unit)
- 5a:: Inkjet head for front surface (printing unit)
- 5b:: Inkjet head for back surface (printing unit)
- 6a:: Tablet front surface
- 6b:: Tablet back surface
- 7:: Tablet side surface
- 8a:: Tablet delivery part
- 8b:: Tablet delivery part
- 10:: Tablet supply unit
- 11:: Rotation feeder
- 12:: Casing
- 13:: Rotary disk
- 14:: Annular rotary plate
- 15:: Communication part
- 16:: Guide plate
- 17:: Tablet acquisition part
- 18:: Suction hole (suction part)
- 19:: Recessed part
- 21:: Rotary shaft
- 22:: Tablet supplying part
- 23:: Tablet feeding part
- 24:: Rotary shaft
- 25:: Suction hole (suction part)
- 26:: Side surface inspection device
- 27:: Rotary shaft
- 28:: Suction hole (suction part)
- 29:: Print surface inspection device
- 31:: Powder removing device
- 32:: Nozzle
- 33:: Suction tube
- 34:: Print inspection device
- 35:: Defective discharge part
- 36:: Jet nozzle
- 37:: Drying/cooling device
- 38:: Good product discharge part
- 39:: Carrying-out path
- 41:: Heating nozzle
- 42:: Cooling nozzle
- 51:: First layer (visible layer)
- 52:: Second layer (invisible layer)
- 53:: Package
- 54:: Inspection device
- 55:: data collating device
- 56:: ROM
- 57:: RAM
- 58:: Data collating part
- 59:: Display device
- A:: Conveying disk (tablet supply disk)
- B:: Conveying disk (first conveying disk: conveying unit)
- C:: Conveying disk (second conveying disk: conveying unit)
- Dt:: Tablet maximum diameter
- W:: Disk end surface width
- Xa:: Disk end surface (tablet holding part)
- Xb:: Disk end surface (tablet holding part)
- Xc:: Disk end surface (tablet holding part)
- Ya:: Disk surface
- Yb:: Disk surface
- Yc:: Disk surface

## Claims

1. A tablet **characterized by** comprising:
a first layer printed using visible ink; and
a second layer printed using invisible ink.

2. The tablet according to claim 1, **characterized in that** the first and second layers are disposed so as to overlap each other.

3. The tablet according to claim 1 or 2, **characterized in that**
the first and second layers are formed at least one of the front and back surfaces of the tablet.

4. A tablet printing device **characterized by** comprising:
a conveying unit conveying a tablet while sucking and supporting the side surface of the tablet; and
a printing unit disposed in proximity to the conveying unit and capable of applying print processing at least to one of front and back surfaces of the tablet conveyed by the conveying unit, wherein
the printing unit forms a first layer printed using visible ink and a second layer printed using invisible ink on the tablet.

5. The tablet printing device according to claim 4, **characterized in that**
the conveying unit is a conveying disk formed into a disk shape, and
the conveying disk has suction parts formed in the end surface thereof in the peripheral direction and each sucking the side surface of the tablet.

6. A tablet printing method **characterized by** comprising:
conveying a tablet while sucking and supporting the side surface of the tablet; and
forming a first layer printed using visible ink and a second layer printed using invisible ink at least on one of the front and back surfaces of the conveyed tablet.
